# FASCICULE DE BREVET EUROPEEN

(11) **EP 3 085 418 B1**
(45) Date de publication et mention de la délivrance du brevet: **12.07.2017**
(21) Numéro de dépôt: 16166002.2
(22) Date de dépôt: 19.04.2016
(51) Int. Cl.: A61Q 19/00, A61Q 19/08, A61K 8/73, A61K 8/97

(54) **COMPOSITIONS COSMETIQUES COMPRENANT DES OLIGOMERES D'ACIDE HYALURONIQUE ET DES CELLULES VEGETALES DEDIFFERENCIEES ET ELICITEES DE BOUGAINVILLIER ENCAPSULANT UN EXTRAIT DE SAFRAN**
KOSMETISCHE ZUSAMMENSETZUNGEN, DIE OLIGOMERE DER HYALURONSÄURE UND DEDIFFERENZIERTE, AUS BOUGAINVILLEA EXTRAHIERTE PFLANZLICHE ZELLEN BEINHALTEN, DIE EIN SAFRANEXTRAKT EINKAPSELN
COSMETIC COMPOSITIONS COMPRISING HYALURONIC ACID OLIGOMERS AND PLANT CELLS FROM BOUGAINVILLAEA WHICH ARE DEDIFFERENTIATED AND ELICITED ENCAPSULATING A SAFFRON EXTRACT

(30) Priorité: 20.04.2015 FR 1553493
(43) Date de publication de la demande: 26.10.2016
(73) Titulaire: Société De Recherche Cosmétique S.à.r.L., 2314 Luxembourg (LU)
(72) Inventeur: LECONTE, Nadine, 92130 ISSY LES MOULINEAUX (FR); FIORENTINO, Julia, 75014 PARIS (FR)
(74) Mandataire: Goulard, Sophie

(56) Documents cités:
- EP-A1- 1 310 262
- WO-A1-03/077880
- WO-A2-03/077881
- WO-A2-2004/082643
- WO-A2-2008/148967
- FR-A1- 2 949 975
- US-A1- 2009 208 544
- CHEN N ET AL: "Hyaluronic acid production promoter for use in topical preparation or cosmetics for moisturizing skin and preventing aging, contains plants e.g. Solena amplexicaulis, Laggera alata and/or Kalimeris indica, or its extract", WPI / THOMSON,, vol. 2007, no. 30, 5 avril 2007 (2007-04-05), XP002733987,

## Description

La présente invention se rapporte au domaine technique général des produits cosmétiques à usage topique, utilisables notamment sur la peau.

Plus précisément, la présente invention est relative à des compositions cosmétiques à application topique comprenant des oligomères d'acide hyaluronique et des cellules végétales dédifférenciées et élicitées de Bougainvillier encapsulant un extrait de safran. Elle se rapporte également à un procédé cosmétique non thérapeutique pour les soins de la peau mettant en oeuvre une telle composition ainsi qu'à l'utilisation non thérapeutique d'une telle composition cosmétique pour le soin de la peau, et notamment pour améliorer la fermeté de la peau, notamment en favorisant la régénération du derme, et pour réduire les rides cutanées.

La peau est un véritable organe comprenant plusieurs couches intégrées, allant de la couche superficielle, l'épiderme, jusqu'aux couches plus profondes, le derme et l'hypoderme, et chacune de ces couches possède des propriétés spécifiques permettant à l'ensemble de réagir et de s'adapter aux conditions de son environnement et de conférer à la peau sa souplesse et sa résistance.

L'épiderme, principalement composé de kératinocytes, de mélanocytes et des cellules de Langerhans, joue un rôle fondamental pour assurer la protection et le maintien d'une bonne trophicité. Les kératinocytes sont des cellules extrêmement dynamiques qui subissent une prolifération et une différenciation permanentes aux termes desquelles elles se transforment en cellules mortes (cornéocytes), s'éliminant régulièrement par desquamation.

Le derme se compose principalement de collagène, d'élastine et de protéoglycanes, molécules synthétisées par les fibroblastes dermiques. Les fibres de collagène assurent la résistance mécanique et la texture de la peau, l'élastine est responsable de l'élasticité, et les protéoglycanes, combinant protéines et glycosaminoglycanes, jouent un rôle majeur de structure et d'hydratation de la peau. Les glycosaminoglycanes, tels que l'acide hyaluronique, sont des molécules très hygroscopiques qui sont capables de retenir des quantités importantes d'eau. D'autres cellules comme les macrophages et les leucocytes sont également présentes dans la couche du derme.

L'hypoderme, qui est la couche la plus profonde de la peau, contient les adipocytes qui produisent des lipides pour que le tissu sous-cutané fabrique une couche grasse protégeant les muscles, les os et les organes internes contre les chocs.

Le collagène est une protéine de structure majeure qui représente plus de 70% de la quantité de protéines de la peau. 14 types de collagène sont décrits dans la peau humaine. Le collagène I est le principal et représente 90% du collagène total dans la peau.

Au cours du temps, de profondes modifications se produisent au niveau du derme. La population de fibroblastes, cellules spécialisées dans la synthèse des fibres d'élastine et de collagène, va diminuer de moitié entre 20 et 80 ans. Ces fibroblastes assurent l'équilibre entre la synthèse, la maturation et la dégradation des fibres d'élastine et de collagène. Dans le temps, l'équilibre va se déplacer vers une dégradation de ces fibres avec pour résultats, une perte d'élasticité et de tonicité du derme et une flaccidité qui ne s'oppose plus aux effets de contraction des muscles sous-jacents, conduisant à l'apparition de rides. Ainsi, avec le vieillissement, le derme subit un amincissement et une rigidification : la peau est moins souple, moins ferme avec l'âge et des rides apparaissent. De plus, la peau doit continuellement faire face à de multiples agressions extérieures qui peuvent accélérer le processus naturel du vieillissement.

Ainsi, de nouveaux actifs cosmétiques sont constamment recherchés afin de lutter contre les signes cutanés du vieillissement et, en particulier, contre l'atrophie du derme et l'apparition de rides.

Les cellules élicitées et dédifférenciées de culture végétale de Bougainvillier sont décrites dans la demande de brevet FR 14 53647. Ces cellules permettent de véhiculer tout type d'ingrédient actif et de les libérer de façon progressive au coeur des différentes couches de l'épiderme, que ces ingrédients actifs soit hydrosolubles ou liposolubles. En effet, les cellules dédifférenciées et élicitées de Bougainvillier se présentent sous la forme de vésicules comprenant une membrane végétale formée d'une bicouche lipidique délimitant un volume interne renfermant une phase encapsulée. Les constituants de la membrane sont identiques aux lipides inter-cornéocytaires de la couche supérieure de l'épiderme et aux lipides intercellulaires (acides gras, céramides, cholestérol). Ainsi, après application des cellules dédifférenciées et élicitées, le contenu cellulaire va diffuser par simple cisaillement de la peau ; le ou les ingrédients actifs bénéficient d'une encapsulation naturelle et d'une libération par bio-mimétisme. Ainsi, après application sur la peau, l'action des cellules s'effectue en 3 temps qui sont 1) la fragmentation des membranes lipidiques des cellules entières, 2) l'insertion dans les lipides inter-cornéocytaires et 3) la libération progressive du ou des ingrédients actifs par bio-mimétisme au sein même de l'épiderme. Il a ainsi été démontré, que l'encapsulation d'un extrait de safran, en tant qu'ingrédient actif, par les cellules dédifférenciées et élicitées de Bougainvillier a un effet positif sur la prolifération des cellules du derme et de l'épiderme de la peau, favorisant ainsi leur régénération.

Toutefois, si cette composition a un effet favorable sur l'abondance du collagène I et la régénération du derme, la nécessité de proposer de nouveaux ingrédients actifs permettant de lutter plus efficacement contre les signes du vieillissement cutané et, en particulier, contre la perte de fermeté de la peau et l'apparition de rides, demeure.

L'acide hyaluronique est un glycosaminoglycane présent chez tous les vertébrés, principalement dans les tissus conjonctifs, épithéliaux et nerveux. C'est l'un des principaux composants de la matrice extracellulaire. L'acide hyaluronique est un constituant naturel du derme possédant un rôle important dans l'hydratation, la tonicité et l'élasticité de la peau. C'est dans les organismes jeunes qu'on le trouve en plus grande quantité. En effet, avec le temps, sa présence diminue, en particulier parce que les radicaux libres auxquels nous sommes exposés (soleil, pollution...) le détruisent.

En cosmétique, les oligomères d'acide hyaluronique de bas poids moléculaire (i.e. généralement inférieur à 10 kDa) jouent un rôle important dans la migration et la prolifération des cellules, deux processus qui interviennent lors de toute régénération, reconstruction, ou cicatrisation des plaies. Les capacités régénérantes des oligomères de bas poids moléculaire peuvent donc être efficacement utilisées, pour traiter le vieillissement de la peau.

Les travaux réalisés par la Demanderesse ont montré que des oligomères d'acide hyaluronique de bas poids moléculaire, i.e. inférieur à 10 kDa potentialisaient l'effet des cellules dédifférenciées et élicitées de Bougainvillier encapsulant un extrait de safran sur la prolifération des cellules du derme, plus particulièrement sur la synthèse du collagène I, favorisant ainsi sa régénération ; L'association d'oligomères d'acide hyaluronique de bas poids moléculaire, i.e. inférieur à 10 kDa et de cellules dédifférenciées et élicitées de Bougainvillier encapsulant un extrait de safran peut donc avantageusement être mise à profit pour le soin de la peau, et en particulier pour favoriser la régénération du derme. De plus, aux doses utilisées, les essais effectués ont montré que les compositions cométiques à base de cellules dédifférenciées de Bougainvillier utilisées dans la présente invention ne présentent aucune cytotoxicité.

La présente invention a donc pour premier objet une composition cosmétique comprenant des cellules végétales dédifférenciées et élicitées de Bougainvillier se présentant sous forme de vésicules, lesdites vésicules comprenant une membrane végétale formée d'une bicouche lipidique délimitant un volume interne renfermant une phase encapsulée, ladite composition étant caractérisée en ce qu'elle comprend en outre au moins un oligomère d'acide hyaluronique de poids moléculaire inférieur à 10 kDa et en ce qu'un extrait de safran est présent dans la phase encapsulée des vésicules.

Selon l'invention, la composition cosmétique est de préférence une composition cosmétique à application topique.

L'invention a également pour autre objet un procédé cosmétique non thérapeutique pour les soins de la peau, en particulier pour améliorer la fermeté de la peau, en favorisant la régénération du derme, et pour réduire les rides cutanées, caractérisé en ce qu'il consiste à appliquer sur les zones de la peau concernées au moins une composition cosmétique telle que définie ci-dessus, c'est-à-dire une composition cosmétique comprenant au moins un oligomère d'acide hyaluronique de poids moléculaire inférieur à 10 kDa et des cellules végétales dédifférenciées et élicitées de Bougainvillier se présentant sous forme de vésicules dont la phase encapsulée comprend au moins un extrait de safran. Enfin, l'invention a pour objet l'utilisation non thérapeutique d'une composition cosmétique à application topique comprenant au moins un oligomère d'acide hyaluronique de poids moléculaire inférieur à 10 kDa et des cellules végétales dédifférenciées et élicitées de Bougainvillier encapsulant un extrait de safran, pour les soins de la peau, en particulier pour améliorer la fermeté de la peau, notamment en favorisant la régénération du derme de la peau, et pour réduire les rides cutanées.

Toutes les espèces de Bougainvillier peuvent être utilisées selon la présente invention, mais on peut plus particulièrement mentionner *Bougainvillea glabra, Bougainvillea alba, Bougainvillea spectabilis, Bougainvillea infesta,* et *Bougainvillea buttiana.*

Par cellules végétales dédifférenciées de Bougainvillier, on entend toute cellule végétale issue d'une culture *in vitro* de cellules de Bougainvillier, lesdites cellules ne présentant aucun des caractères d'une spécialisation particulière et étant capables de vivre par elles-mêmes et non en dépendance avec d'autres cellules.

Les cellules dédifférenciées et élicitées de Bougainvillier utilisables selon la présente invention peuvent être obtenues par des techniques connues de croissance ou multiplication cellulaire, en milieu liquide ou solide, de cellules de Bougainvillier cultivées *in vitro* dans un environnement contrôlé, dans des conditions aseptiques en l'absence de micro-organismes.

La technique de culture végétale présente l'avantage de produire des cellules cultivées dans des conditions environnementales bien définies et reproductibles, en ce qui concerne notamment la température, la lumière et le milieu de culture et de produire des cellules non différenciées se trouvant au même stade physiologique à un instant donné (dédifférenciées). Un autre avantage est la formation de métabolites secondaires en un temps plus court, de l'ordre de une à trois semaines, que chez la plante où ce délai est de plusieurs mois.

Par exemple, on peut préparer des cellules dédifférenciées et élicitées de Bougainvillier à partir d'explants de feuilles, de fleurs, de la tige ou de la racine.

Les cellules dédifférenciées et élicitées utilisables selon l'invention peuvent être obtenues par toute méthode connue de l'art antérieur telle que par exemple selon les méthodes décrites par E. F. George et P. D. Sherrington dans Plant Propagation by tissue culture, Handbook and Directory of Commercial Laboratories (Exegetics Ltd, 1984).

Les milieux de culture utilisables selon l'invention sont ceux généralement connus de l'homme du métier. On peut notamment citer à titre d'exemple les milieux de Gamborg, de Murashige Skoog, de Heller, de White, et dont la description complète peut être trouvée dans « Plant Culture Media : formulations and uses », E. F. George, DJM Puttock et H. J. George (Exegetics Ltd 1987, Tomes 1 & 2).

On entend par « élicitation », la mise en oeuvre d'un stress ou d'une agression (biologique, chimique ou physique) sur les cellules végétales dans leur milieu de culture afin de provoquer un ou plusieurs mécanismes de défense se concrétisant notamment, mais non uniquement, par la synthèse de métabolites secondaires d'intérêt tels que les phytoalexines.

Selon l'invention, l'élicitation des cellules dédifférenciées de Bougainvillier en milieu de culture peut être réalisée par application d'agents chimiques ou de stresses divers tels que pression, dépression, vide, variation de pression, présence d'un gaz, atmosphère variable, chaleur, froid, lumière, cycles de luminosité, radiations, toxines, agitation, contamination par des microorganismes (bactéries, levures, virus), ultrasons, rayonnement infrarouge ou ultraviolet, asphyxie. Suivant la présente invention, on peut plus particulièrement utiliser des cellules dédifférenciées, élicitées et lyophilisées de Bougainvilliers. La lyophilisation des cellules permet d'améliorer leur conservation et, par suite, de faciliter leur incorporation dans les compositions cosmétiques.

Les éléments dosés dans les cellules lyophilisées sont les suivants (pour 100 g de cellules lyophilisées) :
**Protéines** (déterminée selon la méthode décrite dans l'Arrêté du 8 septembre 1977 relatif aux méthodes officielles d'analyse des produits diététiques et de régime) : 13,56 g

L'aminogramme de l'extrait protéique a mis en évidence les principaux acides aminés suivants. Dans ce qui suit, les teneurs en acides aminés sont indiquées en grammes pour 100 g de cellules lyophilisées.

| | |
|---|---|
| Acide aspartique | 10,61 |
| Acide glutamique | 10,15 |
| Proline | 6,75 |
| Lysine | 6,38 |
| Valine | 5,79 |
| Alanine | 5,29 |
| Sérine | 5,27 |
| Glycine | 4,82 |
| Arginine | 4,72 |
| Glutamine | 4,63 |
| Isoleucine | 4,61 |
| Leucine | 4,60 |
| Phénylalanine | 4,25 |
| Thréonine | 4,01 |
| Tyrosine | 3,70 |
| Asparagine | 3,29 |
| Tryptophane | 3,02 |
| Méthionine | 2,58 |
| Cystéine | 1,89 |
| Ornithine | 0,24 |

**Lipides** (déterminés selon la méthode décrite dans l'Arrêté du 8 septembre 1977 relatif aux méthodes officielles d'analyse des produits diététiques et de régime) : 1,08 g, se composant notamment de 73 % en masse environ d'acides gras saturés et insaturés en C₁₈ et de 21 % en masse environ d'acides gras saturés en C₁₆.

**Sucres** (déterminés par Chromatographie ionique - Détection ampérométrique pulsée (PAD)) :

| | |
|---|---|
| - Glucose | 11,23 g |
| - Fructose | 13,10 g |
| - Lactose | < 0,12 g |
| - Saccharose | 10,78 g |
| - Maltose | < 0,13 g |

Les cellules élicitées et dédifférenciées de Bougainvillier selon la présente invention encapsulent un extrait de fleurs de safran *(Crocus sativus*) à titre d'ingrédient actif. L'extrait de safran peut être obtenu à partir de fleurs de safran, après prélèvement des stigmates, et des pistils. Les fleurs sans stigmates et les pistils sont ensuite séchés, réduits en poudre puis extraits à l'eau par macération.

Ainsi, les cellules dédifférenciées et élicitées de Bougainvillier encapsulant un extrait de safran sont de préférence obtenues par le procédé comprenant les étapes suivantes :
1) La préparation de cellules dédifférenciées par culture *in vitro* en milieu de culture d'explants de Bougainvillier,
2) L'élicitation des cellules dédifférenciées obtenues à l'étape précédente dans le milieu de culture,
3) L'extraction des cellules dédifférenciées et élicitées obtenues ci-dessus à l'étape précédente,
4) L'incubation des cellules dédifférenciées et élicitées obtenues à l'étape 3) avec une solution ou une dispersion contenant l'extrait de safran dans le milieu liquide.

L'étape 4) d'incubation est de préférence réalisée à une température inférieure ou égale à 30°C, et encore plus préférentiellement à une température variant de 20 à 30°C, pendant une durée de 12 à 48 heures environ. Le milieu liquide utilisable pour effectuer l'incubation des cellules dédifférenciées et élicitées avec le ou les ingrédients actifs peut par exemple être choisi parmi les milieux utilisés à l'étape 1) du procédé pour la culture *in vitro* des cellules, c'est à dire pour la multiplication cellulaire des cellules de Bougainvillier.

A la fin de l'incubation, les cellules dédifférenciées et élicitées de Bougainvillier encapsulant l'extrait de safran peuvent être récupérées dans le milieu liquide, par exemple par filtration.

La quantité d'extrait de safran présente dans la phase encapsulée varie généralement de 0,01 à 5% en masse environ par rapport à la masse totale des cellules dédifférenciées et élicitées de Bougainvillier, et encore plus préférentiellement de 0,1 à 3% en masse environ. De manière tout particulièrement préférée, la quantité d'extrait de safran présente dans la phase encapsulée est de 1% en masse environ.

Selon l'invention, la phase encapsulée est de préférence une phase aqueuse choisie parmi l'eau et les milieux de culture utilisés pour la culture in vitro desdites cellules.

Selon une forme particulièrement avantageuse de l'invention, les cellules dédifférenciées et élicitées de Bougainvillier encapsulant l'extrait de safran sont dispersées dans la glycérine pour obtenir une suspension de cellules, avant d'être incorporées dans une composition cosmétique. Ainsi, selon une forme de réalisation préférée de l'invention, les cellules dédifférenciées et élicitées encapsulant un extrait de safran sont présentes sous la forme d'une suspension desdites cellules dans la glycérine. De préférence, la quantité de cellules dédifférenciées et élicitées encapsulant un extrait de safran au sein de ladite suspension varie de 15 à 30% en masse environ, et encore plus préférentiellement est de l'ordre de 20% en masse environ par rapport à la masse totale de ladite suspension.

Ainsi, selon cette forme particulière de réalisation de l'invention, la composition cosmétique peut alors comprendre de 0,01 à 10% en masse environ d'une suspension de cellules végétales dédifférenciées et élicitées de Bougainvillier encapsulant un extrait de safran dans la glycérine et telle que décrite ci-dessus, par rapport à la masse totale de la composition cosmétique, de préférence de 0,05% à 5% en masse environ, et encore plus préférentiellement de 0,1% à 3% en masse environ.

Selon l'invention, le ou les oligomères d'acide hyaluronique de poids moléculaire inférieur à 10 kDA répondent de préférence à la formule (C₁₄H₂₁NO₁₁)ₙ, dans laquelle n est un nombre entier inférieur à 25 désignant le degré de polymérisation.

Selon une forme de réalisation préférée de l'invention, n est compris entre 5 et 25.

De façon avantageuse, la concentration en oligomères d'acide hyaluronique de poids moléculaire inférieur à 10 kDA dans la composition cosmétique de l'invention peut varier de 0,01% à 1% environ, de préférence de 0,05% à 0,5% environ, et encore plus préférentiellement de 0,1 à 0,3% environ en masse par rapport à la masse totale de la composition cosmétique.

Les oligomères d'acide hyaluronique de poids moléculaire inférieur à 10 kDA utilisables dans le cadre de l'invention peuvent être obtenus grâce à la production d'acide hyaluronique selon des procédés biotechnologiques bien connus de l'homme du métier et tels que décrit par exemple par Schiraldi C. et al. (« Biotechnological Production and Application of Hyaluronan », dans Biopolymers, M. Elnashar, Ed., pp. 387-412, InTech, Rijeka, Croatia, 2010) puis hydrolyse de l'acide hyaluronique afin d'obtenir des oligomères d'acide hyaluronique de poids moléculaire inférieur à 10 kDa.

Les méthodes de mesure du poids moléculaire des oligomères d'acide hyaluronique utilisables selon l'invention sont celles généralement connues de l'homme du métier, et tels que décrites par exemple par Mahnoey D.J. et al (« Novel methods for the preparation and characterization of hyaluronan oligosaccharides of defined lengh », Glycobiology. 2001, 11(12), 1025-1033).

On peut notamment citer à titre d'exemple la combinaison de diffusion de la lumières aux angles multiples couplée à une chromatographie d'exclusion sérique (MALLS-SEC) qui sépare les protéines suivant leur volume hydrodynamique et permet d'avoir accès à la masse moléculaire des espèces et à l'homogénéité d'un pic en sortie de chromatographie d'exclusion sérique. Le poids moléculaire des oligomères d'acide hyaluronique de formule (C₁₄H₂₁NO₁₁)n, dans laquelle n est un nombre entier inférieur à 25 selon la présente invention varie ainsi entre 2 kDa inclusivement et 10kDaltons exclusivement.

Des oligomères d'acide hyaluronique de poids moléculaire inférieur à 10 kDA sont également disponibles dans le commerce. A titre d'exemple, on peut notamment mentionner les produits vendus sous les dénominations commerciales Signal-10 ® (CAS Number 90004-61-9) par la société Principium S.A. (Viganello, CH), ou PrimaHyal 10 ® par la société Soliance (Pomacle, FR).

Les compositions suivant la présente invention comprennent en association au moins un oligomère d'acide hyaluronique de poids moléculaire inférieur à 10 kDA et les cellules végétales élicitées et dédifférenciées de Bougainvillier encapsulant un extrait de safran, en combinaison, le cas échéant avec les supports et excipients usuels dans les compositions cométiques et dermatologiques, cosmétiquement et dermatologiquement acceptables.

Au sens de la présente invention, on entend par « support cosmétiquement et dermatologiquement acceptable », les milieux comprenant de l'eau, un mélange d'eau et d'un ou plusieurs solvants organiques, ou un solvant ou un mélange de solvants organiques cosmétiquement ou dermatologiquement acceptables.

Suivant une forme avantageuse de réalisation, les compositions cométiques conformes à la présente invention peuvent contenir, outre les cellules végétales dédifférenciées et élicitées de Bougainvillier encapsulant un extrait de safran et les oligomères d'acide hyaluronique de poids moléculaire inférieur à 10 kDa, un ou plusieurs actifs supplémentaires renforçant ou complétant avantageusement leur activité, et compatibles, c'est-à-dire non susceptibles de réagir les uns avec les autres ou de masquer ou limiter leurs effets. De tels ingrédients actifs peuvent par exemple être choisis parmi tout type de composés habituellement utilisés dans les compositions cosmétiques. A titre d'exemple, on peut citer les extraits de plancton, les extraits d'origine végétale tels qu'un extrait de maca (qui a un effet sur la prévention de dérèglements liés à une déficience de la microcirculation cutanée et au stress oxydatif qui peut en résulter), un extrait de pétales de coquelicot agissant sur la nutrition cellulaire, une combinaison d'extraits d'anchuse, de coquelicot et de passiflore qui, en combinaison, ont un effet dans la prévention des signes du vieillissement cutané, un extrait de graines de mimosa, un extrait de pétales de souci, un extrait de barbitamao, un extrait de kigelia africana, un extrait d'avoine. Les compositions cosmétiques conformes à la présente invention peuvent se présenter sous les formes galéniques classiquement utilisées pour une application par voie topique, c'est-à-dire, sous forme de gel, d'émulsion (en particulier crème ou lait), d'émulsion biphasique huile-dans-eau ou eau-dans-huile, d'huile corporelle, de masque, de pommade, d'onguent, de lotion, de solution concentrée, lesdites formes galéniques contenant en outre des excipients et supports usuels et acceptables sur le plan cosmétologique. Elles sont utilisées de préférence sous forme de crèmes, de sérum, de lotion ou de gel.

Ces formes d'administration par voie topique sont préparées par les techniques usuelles, et par exemple, dans le cas d'une crème, par dispersion d'une phase grasse dans une phase aqueuse pour obtenir une émulsion huile-dans-eau, ou inversement pour préparer une émulsion eau-dans-huile dans les cas de crèmes, on peut utiliser des émulsions à structure lamellaire obtenues avec des lécithines hydrogénées ou des sucro-esters, contenant peu de produits éthoxylés ou n'en contenant pas du tout.

Les compositions topiques selon l'invention peuvent comprendre des excipients appropriés pour une application topique externe, en particulier des excipients acceptables sur le plan cosmétologique. Ces excipients appropriés pour la formulation sont bien connus de l'homme du métier et comprennent en particulier des agents de pénétration tels que le phytantriol, l'octyl dodécanol et l'escine ; les épaississants tels que les gommes naturelles et les polymères de synthèse ; les tensioactifs ; les émollients tels que l'octanoate de cétéaryle, le myristate d'isopropyle, l'isononanoate de cétéaryle, la diméthicone, la cyclométhicone, le 3-diisostéarate de polyglycéryle, le polyisobutène hydrogéné, l'alcool cétylique, le palmitate cétylique, le phosphate cétylique ; les émulsionnants tels que des dérivés de polyglycérol ; les conservateurs tels que le phénoxyéthanol et l'acide déhydroacétique ; les colorants ; les parfums. Comme autres ingrédients utilisables dans les compositions de l'invention, on peut citer les agents hydratants tels que la glycérine, le butylène glycol, le sel de sodium de l'acide pyrrolidone carboxylique (sodium PCA), ainsi que des associations de dérivés glucosiques à effet hydratant et restructurant comme le produit Aquaxyl® (xylitylglucoside et anhydroxylytol et xylitol), et également les vitamines antioxydantes.

On peut aussi ajouter à la composition des glucides choisis principalement parmi le glucose, le glycogène et le tréhalose, ou encore un palmitoyl pentapeptide-3 tel que le Matrixyl® ou des dérivés tels que le palmitoyl GHK (possédant la chaîne Glycyl-Histidyl-Lysine) et le palmitoyl GQPR (Glycyl-Glutamyl-Prolyl-Arginine) ou le palmitoyl VGVAPG (Valyl-Glycyl-Valyl-Alanyl-Prolyl-Glycine) associé à un céramide-2, ainsi que d'une manière générale toute association avec un céramide.

On peut également ajouter à la composition des agents de protection contre les rayons ultraviolets, et par exemple des filtres solaires UV-A et UV-B hydrophiles ou lipophiles, ou des pigments formant écran anti-ultraviolet.

Les filtres solaires peuvent être choisis par exemple parmi la benzophénone ou un dérivé de benzophénone tel que la 2-hydroxy-4-méthoxy-benzophénone (Eusolex® 4360), ou un ester d'acide cinnamique et plus particulièrement le méthoxycinnamate d'octyle (Eusolex® 2292), le méthoxycinnamate d'éthyl-2-hexyle (Parsol MCX®), ou encore un cyano-β,β-diphénylacrylate tel que l'octocrylène (Eusolex® OCR), le 4-méthylbenzylidène camphre (Eusolex 6300®), et des dérivés du dibenzoylméthane tels que le 4-isopropyl dibenzoylméthane (Eusolex 8020), le t-butyl-méthoxy dibenzoylméthane (Parsol 1789®), et le 4-méthoxy-dibenzoylméthane.

Les pigments peuvent être choisis par exemple parmi le dioxyde de titane, l'oxyde de zinc et l'oxyde de zirconium.

L'association, selon la présente invention, de cellules dédifférenciées et élicitées de Bougainvillier encapsulant un extrait de safran et d'oligomères d'acide hyaluronique de poids moléculaire inférieur à 10 kDa a fait apparaitre une excellente activité sur la synthèse du collagène I. Ainsi aux concentrations testées, on a pu constater une augmentation hautement significative (+106%) de l'abondance du collagène I. Ces résultats confirment que l'association selon l'invention favorise la régénération du derme et permet d'améliorer la fermeté de la peau et de réduire l'apparition de rides.

L'utilisation non thérapeutique de la composition cosmétique conforme à l'invention comprend tous les soins du corps et de la peau y compris les produits solaires, protecteurs et bronzants, les produits anti-âges, anti-séborrhéiques, toniques, les produits assurant l'amélioration de l'aspect de la peau y compris le traitement acnéique et les rougeurs cutanées.

Les résultats sont explicités dans les exemples ci-après et démontrent la synergie des effets de chacun des composants, puisque, à doses équivalentes, les résultats procurés par l'association sont supérieurs à la somme des effets qu'elles produisent individuellement.

Les exemples suivants illustrent l'invention sans pour autant en limiter la portée.

### EXEMPLES

### EXEMPLE 1 : Préparation d'une suspension de cellules végétales dédifférenciées et élicitées de Bougainvillier encapsulant un extrait de safran

### 1. Prélèvement et culture des cellules dédifférenciées de Bougainvillier

Des morceaux de feuilles d'environ 5 à 10 mm ont été prélevés sur des feuilles fraîches de bougainvillier (*Bougainvillea glabra*).

Les morceaux de feuilles ont ensuite été stérilisés, puis mis en culture dans des boites stériles à température ambiante sur un milieu nutritif synthétique (milieu de Murashige et Skoog gélosé), jusqu'à formation, au niveau des explants, d'amas cellulaire dédifférenciés, appelés cals ou cellules mères.

Les cellules mères ont été prélevées au bout de 30 jours, puis repiquées sur un milieu nutritif neuf dans les mêmes conditions de culture que précédemment jusqu'à l'obtention de cals friables.

On a ainsi obtenu une collection de souches dédifférenciées stables présentant des caractéristiques de croissance et de production de métabolites.

### 2. Elicitation des cellules obtenues ci-dessus à l'étape précédente

L'élicitation des cellules a été réalisée par irradiation par la lumière UV à 180 nm pendant 2 heures, à l'aide d'une lampe Wilbert-Lourmat T-30C (600µW/m²), positionnée à une distance de 1 m en éclairage direct au-dessus des cellules.

### 3. Préparation de la suspension cellulaire

Après élicitation, un morceau de cal a été prélevé puis mis en suspension dans un milieu nutritif liquide (milieu de Murashige et Skoog sans gélose).

A la fin de la culture (15 jours), les cellules ont été filtrées pour éliminer le milieu de culture puis rincées à l'eau froide (4°C). On a ainsi obtenu une biomasse fraiche d'environ 250 g de cellules par litre de culture.

### 4. Obtention de l'extrait de safran

L'extrait de safran a été obtenu à partir de fleurs de safran, après prélèvement des stigmates, et des pistils. Les fleurs sans stigmates et les pistils ont été séchés, réduits en poudres puis extraits à l'eau par macération.

### 5. Encapsulation de l'extrait de safran dans les cellules dédifférenciées et élicitées de Bougainvillier

Des cellules dédifférenciées et élicitées de Bougainvillier telles que préparées ci-dessus ont été mises en suspension dans du milieu nutritif liquide (milieu de Murashige et Skoog sans gélose) contenant 1% en masse d'extrait de safran. L'incubation des cellules de Bougainvillier a été réalisée pendant 48 heures, à une température de 25°C afin d'encapsuler l'extrait de safran dans la phase aqueuse des cellules.

A la fin de la période d'incubation, les cellules de Bougainvilliers ont été récupérées par filtration puis mises en suspension dans la glycérine à raison de 20% en masse pour 80% en masse de glycérine.

On a ainsi obtenu une suspension comprenant 80% en masse de glycérine, 20% en masse de cellules dédifférenciées et élicitées de Bougainvillier encapsulant un extrait de safran.

### EXEMPLE 2 : Mise en évidence de l'absence de cytotoxicité de l'association des cellules végétales de Bougainvillier encapsulant un extrait de safran et d'oligomères d'acide hyaluronique de poids moléculaire inférieur à 10 kDa

Dans cet exemple, on a testé la cytotoxicité éventuelle des cellules dédifférenciées et élicitées de Bougainvillier encapsulant un extrait de safran telles qu'obtenues à l'exemple 1, d'oligomères d'acide hyaluronique de formule (C₁₄H₂₁NO₁₁)n, où n est un nombre entier inférieur à 25 vendus sous la dénomination commerciale Signal-10 par la société Principium S.A., ainsi que de leur association.

L'étude a été réalisée sur des fibroblastes de derme humains NHDFs (Normal Human Dermal Fibroblasts ; origine : prépuce) à environ 40% de leur potentiel prolifératif et cultivés en monocouche en milieu DMEM (Dulbecco's Modified Eagle Medium) contenant du sérum FBS (Fetal Bovine Serum) et des antibiotiques (pénicilline/streptomycine).

Les différents traitements avec les actifs et leur association ont été réalisés dans du milieu de culture ne contenant pas de sérum.

Les cellules ont été maintenues dans une atmosphère humide à 37°C contenant 5% De CO₂.

Les cellules ont été ensemencées en plaque 24 puits, 24 heures avant le traitement avec respectivement 0,3% d'oligomères d'acide hyaluronique de formule (C₁₄H₂₁NO₁₁)n, où n est un nombre entier inférieur à 25, 3% de cellules dédifférenciées et élicitées de Bougainvillier encapsulant un extrait de safran, et l'association de ces deux actifs à ces deux concentrations.

Le traitement des fibroblastes NHDFs par les deux actifs et leur association, appliqués à ces concentrations et en triplicat de culture (n=3), a été réalisé dans le milieu de culture DMEM contenant des antibiotiques et en absence de sérum.

La viabilité des cellules a ensuite été analysée par utilisation du test MTS (3-(4,5-diméthylthiazol-2-yl)-5-3(3-carboxy-méthoxyphényl)-2-(4-sulfophényl)-2H-tetrazolium) après 48 heures de traitement.

La densité cellulaire et la cinétique expérimentale correspondent aux conditions qui ont été utilisées par la suite pour l'analyse de l'effet des actifs sur l'abondance du collagène I.

Une solution aqueuse de dodécyl sulfate de sodium à 0,08 % en masse (SDS) a été utilisée en tant que contrôle positif, afin de valider l'expérience de cytotoxicité.

Le seuil de cytotoxicité a été arbitrairement fixé à 80% de viabilité par rapport au contrôle non traité et le SDS a été utilisé comme contrôle positif de cytotoxicité afin de valider l'expérience.

Il n'existe pas de différence significative concernant le pourcentage de viabilité des fibroblastes NHDFs après 48 heures de traitement entre les cellules traitées par les deux actifs ou leur association et les cellules témoins n'ayant reçu aucun traitement (résultats non présentés).

Ces résultats sont significatifs d'une absence de cytoxicité.

### EXEMPLE 3 : Evaluation de l'effet de l'association des cellules dédifférenciées et élicitées de Bougainvillier encapsulant un extrait de safran et d'oligomères d'acide hyaluronique de poids moléculaire inférieur à 10 kDa sur la régénération du derme

Le test est basé sur l'évaluation par immunomarquage en fluorescence du collagène I afin d'estimer l'effet des cellules dédifférenciées et élicitées encapsulant un extrait de safran, d'un oligomère de poids moléculaire inférieur à 10kDa, ainsi que de leur association sur la synthèse de collagène I.

Afin d'évaluer les effets sur la synthèse du collagène I, les fibroblastes NHDFs ont été ensemencés sur des lames de verre (compartiment de 0,7 cm²), 24 h avant le début des traitements par les actifs et leur association. Cinq lots ont été constitués :
Lot 1 : Témoin ne recevant aucun produit.
Lot 2 : Contrôle positif. Une solution de TGF-β1 à 20 ng/mL a été utilisée comme molécule de référence permettant d'augmenter la synthèse de collagène I au sein des fibroblastes NHDFs.
Lot 3 : Les fibroblastes ont été traités avec 0.3% d'oligomères d'acide hyaluronique de formule (C₁₄H₂₁NO₁₁)n, où n est un nombre entier inférieur à 25 vendus sous la dénomination commerciale Signal-10 par la société Principium S.A.
Lot 4 : Les fibroblastes ont été traités avec 3% de cellules végétales dédifférenciées et élicitées de Bougainvillier encapsulant un extrait de safran.
Lot 5 : Les fibroblastes ont été traités par l'association de 3% de cellules végétales dédifférenciées et élicitées de Bougainvillier encapsulant un extrait de safran et 0.3% d'oligomères d'acide hyaluronique de formule (C₁₄H₂₁NO₁₁)n, où n est un nombre entier inférieur à 25 vendus sous la dénomination commerciale Signal-10 par la société Principium S.A.

Les actifs ont été solubilisés dans le milieu de culture ne contenant pas de sérum et ont été mis au contact des fibroblastes durant 48 heures. Chaque condition a été réalisée en triplicat (n=3).

Au terme des traitements, les fibroblastes ont été fixés au formol puis rincés au PBS. Les lames ont ensuite été conservées à 4°C jusqu'au jour du marquage.

L'immuno-marquage en fluorescence du collagène I a été réalisé en incubant les lames avec un anticorps primaire spécifique et un anticorps secondaire conjugué à la fluorescéine. Le DAPI (4',6'-diamidino-2-phénylindole), une molécule fluorescente capable de lier l'ADN, a été utilisée pour marquer les noyaux. Les lames ont finalement été montées à l'alcool polyvinylique (produit vendu sous la dénomination commerciale Mowiol® par la société Aldrich) et stockées à 4°C.

La quantification a été réalisée sur la base de 3 photos par réplique prises à l'aide d'un microscope Leica ® (DM 2000, objectif 40x) et d'une caméra Leica ® (DFC420C).

Le marquage en immunofluorescence du collagène I a été quantifié à l'aide du logiciel QWin 3 ® (Leica) en utilisant le pixel comme unité de surface (1 pixel = 1 µm²). Chaque photo est constituée de 4751460 pixels. L'intensité moyenne du marquage a été déterminée pour chaque photo. Les noyaux sont dénombrés grâce au logiciel *ImageJ* afin de rapporter la surface marquée et l'intensité moyenne du marquage au nombre de noyaux sur chaque photo. Ce rapport, permettant de déterminer l'intensité du marquage, est comparé à la valeur correspondant à la condition contrôle non traité. Un test statistique *t-student* a été réalisé afin de comparer l'effet de chaque actif ou de l'association.

Les résultats sont présentés dans le tableau 1 ci-après :

**TABLEAU 1**

| Condition | Marquage en immunofluorescence du collagène I | Différence par rapport au témoin (%) |
|---|---|---|
| Lot 1 | 386497 ± 5 | - |
| Lot 2 | 1 833 434 ± 92 | +374** |
| Lot 3 | 345 167 ± 14 | -11 |
| Lot 4 | 677 881 ± 9 | +75 |
| Lot 5 | 797 444 ± 33 | +106* |

Les valeurs de 0,001<p<0,01 sont considérées comme hautement significatives (*) et p < 0,001 comme très hautement significatives (**) (test t-Student par rapport à la condition CTL non traité)

Ces résultats montrent que le traitement des fibroblastes NHDFs par l'association des cellules végétales dédifférenciées et élicitées de Bougainvillier encapsulant un extrait de safran et des oligomères d'acide hyaluronique de poids moléculaire inférieur à 10 kDa induit une augmentation hautement significative de la synthèse du collagène I (+106% de marquage en immunofluorescence du collagène I par rapport au témoin). Ces résultats sont surprenants, dans la mesure où les oligomères d'acide hyaluronique de poids moléculaire inférieur à 10 kDa n'ont pas d'influence sur la synthèse de collagène I (marquage en immunofluorescence du collagène I diminué de 11% par rapport au témoin). Ainsi, ces résultats démontrent que les oligomères d'acide hyaluronique de poids moléculaire inférieur à 10kDa, potentialisent les effets des cellules végétales dédifférenciées et élicitées de Bougainvillier encapsulant un extrait de safran (augmentation de 106% du marquage en immunofluorescence après traitement par l'association par rapport au témoin vs augmentation de 75% par rapport au témoin, après traitement par les cellules végétales dédifférenciées et élicitées de Bougainvillier encapsulant un extrait de safran).

Ainsi, l'association de cellules végétales dédifférenciées et élicitées de Bougainvillier encapsulant un extrait de safran et d'oligomères d'acide hyaluronique de poids moléculaire inférieur à 10 kDa a un effet supérieur sur la synthèse de collagène I.

### EXEMPLE 4 : Crème pour le soin de la peau

Par les techniques usuelles, on a préparé une crème pour le soin de la peau ayant la composition massique indiquée ci-après :

| | | |
|---|---|---|
| - Gluconate de sodium | 0,2 | g |
| - Copolymère Hydroxyéthyl Acrylate / Sodium acryloyldiméthyl Taurate vendu sous la dénomination Sepinov ® EMT10 par Seppic | 1,65 | g |
| - Glycérine pure à 99,8 % | 2,0 | g |
| - Hydroxyde de sodium | 0,25 | g |
| - Acide citrique, monohydrate | 0,3 | g |
| - Filtres solaires | 15,5 | g |
| - Benzoate d'alkyle en C₁₂-C₁₅ vendu sous la dénomination Cetiol ® AB par BASF | 11,0 | g |
| - Mélange d'alcool arachidylique, d'alcool béhénylique et de glucoside arachidylique vendu sous la dénomination Montanov ® 202 par Seppic | 3,0 | g |
| - Diméthicone vendu sous la dénomination Abil ® 350 par Evonik Industries | 2,0 | g |
| - Huile vierge d'amande douce | 3,0 | g |
| - DL α-tocophérol | 0,5 | g |
| - Hydroxyéthylurée vendue sous la dénomination Hydrovance ® par AkzoNobel | 3,0 | g |
| - Cellules de Bougainvilliers encapsulant un extrait de safran telles que préparées à l'exemple 1 | 1,0 | g |
| - Agent tenseur | 1,0 | g |
| - Extrait hydroglycolique de Crocus décoloré vendu par GREENTECH | 0,3 | g |
| - Cellules de cacao broyées vendues sous la dénomination Ground Cacao cells par DIANA PLANT | 0,01 | g |
| - Oligomères d'acide hyaluronique de poids moléculaire inférieur à 10 kDa vendu sous la dénomination Signal-10 ® par Principium B.S.I. | 0,1 | g |
| - Conservateurs | qs | |
| - Parfums | qs | |
| - Eau déminéralisée 1 | qsp 100,0 | g |

Cette crème anti-âge peut être utilisée une à deux fois par jour par application sur les zones de la peau à traiter.

### EXEMPLE 5 : Sérum pour le soin de la peau

Par les techniques usuelles, on a préparé un sérum pour le soin de la peau ayant la composition massique indiquée ci-après.

| | | |
|---|---|---|
| - Gluconate de sodium | 0,2 | g |
| - Gomme de xanthane vendue sous la dénomination | | |
| - Glycérine pure à 99,8 % | 1,0 | g |
| - Acrylates/C₁₀₋₃₀ Alkyl Acrylate Crosspolymer vendu sous la dénomination Pemulen ® TR-1 par Lubrizol | 0,3 | g |
| - Mélange d'alcools en C₁₄-C₂₀ et d'alkylglycosides en C₁₄-C₂₀ vendu sous la dénomination Montanov ® L par Seppic | 1,5 | g |
| - Stéaroylglutamate de sodium vendu sous la dénomination Eumulgin ® SG par BASF | 0,2 | g |
| - Triglycérides d'acides caprique et caprylique vendus sous la dénomination Myritol ® 318 par BASF | 4,0 | g |
| - Huile vierge d'amande douce | 3,0 | g |
| - DL α-tocophérol | 0,5 | g |
| - Oligomères d'acide hyaluronique de poids moléculaire inférieur à 10 kDa vendu sous la dénomination Signal-10 ® par Principium B.S.I. | 0,1 | g |
| - Cellules de cacao broyées vendues sous la dénomination Ground cells Cacao par DIANA PLANT | 0,01 | g |
| - Hydroxyéthylurée vendue sous la dénomination Hydrovance ® par AkzoNobel | 3,0 | g |
| - Extrait hydroglycolique de Crocus décoloré par la société GREENTECH | 0,3 | g |
| - Cellules de Bougainvilliers encapsulant un extrait de safran telles que préparées à l'exemple 1 | 1,0 | g |
| - Hydroxyde de sodium | 0,07 | g |
| - Conservateurs | qs | |
| - Parfums | qs | |
| - Eau déminéralisée | qsp 100,0 | g |

Ce sérum est destiné à raffermir et régénérer la peau et peut être utilisé une à deux fois par jour.

### EXEMPLE 6 : Emulsion pour le soin de la peau

Par les techniques usuelles, on a préparé une émulsion pour le soin des peaux normales à mixtes ayant la composition massique indiquée ci-après.

| | | |
|---|---|---|
| - Gluconate de sodium | 0,2 | g |
| - Copolymère Hydroxyéthyl Acrylate / Sodium | | |
| acryloyldiméthyl Taurate vendu sous la dénomination Sepinov ® EMT10 par Seppic | 1,2 | g |
| - Glycérine pure à 99,8 % | 1,0 | g |
| - Hydroxyde de sodium | 0,3 | g |
| - Mélange d'alcool arachidylique, d'alcool béhénylique et de glucoside arachidylique vendu sous la dénomination Montanov ® 202 par Seppic | 1,5 | g |
| - Stéaroylglutamate de sodium vendu sous la dénomination Eumulgin ® SG par BASF | 0,25 | g |
| - Palmitate de glycol vendu sous la dénomination Lanol ® P par Seppic | 1,0 | g |
| - Triglycérides d'acides caprique et caprylique vendus sous la dénomination Myritol ® 318 par BASF | 3,0 | g |
| - Polyphénylméthylsiloxane vendu sous la dénomination Dow Corning ® 556 par Dow Corning | 1,0 | g |
| - Huile vierge d'amande douce | 2,0 | g |
| - Huile de Limnanthe | 2,0 | g |
| - DL α-tocophérol | 0,7 | g |
| - Hydroxyéthylurée vendue sous la dénomination Hydrovance ® par AkzoNobel | 3,0 | g |
| - Extrait aqueux de pétales de coquelicots | 0,5 | g |
| - Mélange de butylène glycol, d'extrait d'écorce *d'Enantia Chlorantha* et d'acide oléanolique vendu sous la dénomination commerciale Evermat ® par Sederma | 1,0 | g |
| - Cellules de Bougainvilliers encapsulant un extrait de safran telles que préparées à l'exemple 1 | 1,0 | g |
| - Cellules de cacao broyées vendues sous la dénomination Ground Cacao Cells par DIANA PLANT | 0,01 | g |
| - Oligomères d'acide hyaluronique de poids moléculaire inférieur à 10 kDa vendu sous la dénomination Signal-10 ® par Principium B.S.I. | 0,1 | g |
| - Conservateurs | qs | |
| - Parfums | qs | |
| - Eau déminéralisée | qsp 100,0 | g |

Cette émulsion peut être utilisée une à deux fois par jour par application sur les zones de la peau à traiter.

## Revendications

1. Composition cosmétique comprenant des cellules végétales dédifférenciées et élicitées de Bougainvillier se présentant sous forme de vésicules, lesdites vésicules comprenant une membrane végétale formée d'une bicouche lipidique délimitant un volume interne renfermant une phase encapsulée, ladite composition étant **caractérisée en ce qu'**elle comprend en outre au moins un oligomère d'acide hyaluronique de poids moléculaire inférieur à 10 kDa et **en ce qu'**un extrait de safran est présent dans la phase encapsulée des vésicules.

2. Composition selon la revendication 1, **caractérisée en ce qu'**elle est une composition cosmétique à application topique.

3. Composition cosmétique selon la revendication 1 ou 2, **caractérisée en ce que** la quantité d'extrait de safran présente dans la phase encapsulée varie de 0,01 à 5% en masse par rapport à la masse totale des cellules dédifférenciées et élicitées de Bougainvillier.

4. Composition selon l'une quelconque des revendications précédentes, **caractérisée en ce qu'**elle comprend de 0,01 % à 10 % en masse d'une suspension de cellules végétales dédifférenciées et élicitées de Bougainvillier encapsulant un extrait de safran dans la glycérine, par rapport à la masse totale de ladite composition.

5. Composition selon l'une quelconque des revendications précédentes, **caractérisée en ce que** le ou les oligomères d'acide hyaluronique de poids moléculaire inférieur à 10 kDA répondent à la formule (C₁₄H₂₁NO₁₁)ₙ, dans laquelle n est un nombre entier inférieur à 25 désignant le degré de polymérisation.

6. Composition selon l'une quelconque des revendications précédentes, **caractérisée en ce que** la concentration en oligomères d'acide hyaluronique de poids moléculaire inférieur à 10 kDa varie de 0,01% à 1% en masse par rapport à la masse totale de ladite composition.

7. Composition selon l'une quelconque des revendications précédentes, **caractérisée en ce qu'**elle comprend en outre un ou plusieurs ingrédients actifs choisis parmi les extraits de plancton, un extrait de maca, un extrait de pétales de coquelicot, une combinaison d'extraits d'anchuse, de coquelicot et de passiflore, un extrait de graines de mimosa, un extrait de pétales de souci, un extrait de barbitamao, un extrait de kigelia africana, et un extrait d'avoine.

8. Composition selon l'une quelconque des revendications précédentes **caractérisée en ce qu'**elle se présente sous la forme de gel, d'émulsion, d'émulsion biphasique huile-dans-eau ou eau-dans-huile, d'huile corporelle, de masque, de pommade, d'onguent ou de solution concentrée.

9. Procédé cosmétique non thérapeutique pour les soins de la peau, **caractérisé en ce qu'**il consiste à appliquer sur les zones de la peau concernées au moins une composition cosmétique telle que définie à l'une quelconque des revendications 1 à 8.

10. Utilisation non thérapeutique d'une composition cosmétique telle que définie à l'une quelconque des revendications 1 à 8, pour les soins de la peau.

11. Utilisation non thérapeutique d'une composition cosmétique telle que définie à l'une quelconque des revendications 1 à 8, pour améliorer la fermeté de la peau et réduire les rides cutanées.

## Patentansprüche

1. Kosmetische Zusammensetzung, umfassend dedifferenzierte und aus Bougainvillea extrahierte pflanzlichen Zellen in Form von Vesikeln, wobei die Vesikel eine pflanzliche Membran umfassen, die von eine Lipiddoppelschicht gebildet ist, die ein inneres Volumen begrenzt, welches eine gekapselte Phase einschließt, wobei die Zusammensetzung **dadurch gekennzeichnet ist, dass** sie ferner mindestens ein Oligomer der Hyaluronsäure mit einem Molekulargewicht unter 10 kDa umfasst und dass ein Safranextrakt in der gekapselten Phase der Vesikel vorhanden ist.

2. Zusammensetzung nach Anspruch 1, **dadurch gekennzeichnet, dass** sie eine kosmetische Zusammensetzung für die topische Anwendung ist.

3. Kosmetische Zusammensetzung nach Anspruch 1 oder 2, **dadurch gekennzeichnet, dass** die Menge Safranextrakt, vorhanden in der gekapselten Phase, zwischen 0,01 bis 5 Ma% in Bezug zur Gesamtmasse der dedifferenzierten und aus Bougainvillea extrahierten pflanzlichen Zellen schwankt.

4. Zusammensetzung nach einem der vorangehenden Ansprüche, **dadurch gekennzeichnet, dass** sie 0,01 bis 10 Ma% einer Suspension dedifferenzierter und aus Bougainvillea extrahierter pflanzlicher Zellen umfasst, die einen Safranextrakt in Glycerin einkapseln, in Bezug zur Gesamtmasse der Zusammensetzung.

5. Zusammensetzung nach einem der vorangehenden Ansprüche, **dadurch gekennzeichnet, dass** das oder die Oligomere der Hyaluronsäure mit Molekulargewicht unter 10 kDA der Formel (C₁₄H₂₁NO₁₁)ₙ entsprechen, in welcher n eine Ganzzahl unter 25 ist, die den Polymerisationsgrad bezeichnet.

6. Zusammensetzung nach einem der vorangehenden Ansprüche, **dadurch gekennzeichnet, dass** die Konzentration an Oligomer der Hyaluronsäure mit Molekulargewicht unter 10 kDa von 0,01 bis 1 Ma% in Bezug zur Gesamtmasse der Zusammensetzung schwankt.

7. Zusammensetzung nach einem der vorangehenden Ansprüche, **dadurch gekennzeichnet, dass** sie ferner einen oder mehrere Wirkstoffe umfasst, die aus den Planktonextrakten, einem Macaextrakt, einem Mohnblumenblütenblätterextrakt, einer Kombination von Extrakten von Ochsenzunge, Mohnblume und Passionsblume, einem Mimosensamenextrakt, einem Ringelblumenblütenblätterextrakt, einem Stryphnodendronextrakt, einem Kigelia africana-Extrakt und einem Haferextrakt ausgewählt sind.

8. Zusammensetzung nach einem der vorangehenden Ansprüche, **dadurch gekennzeichnet, dass** sie in Form von Gel, Emulsion, Zweiphasen-Öl-in-Wasser- oder -Wasser-in-Öl-Emusion, Körperöl, Maske, Pomade, Salbe oder konzentrierter Lösung vorliegt.

9. Kosmetisches nicht therapeutisches Verfahren zur Hautpflege, **dadurch gekennzeichnet, dass** es darin besteht, auf die betroffenen Hautbereiche mindestens eine kosmetische Zusammensetzung nach einem der Ansprüche 1 bis 8 aufzutragen.

10. Nicht therapeutische Verwendung einer kosmetischen Zusammensetzung nach einem der Ansprüche 1 bis 8 zur Hautpflege.

11. Nicht therapeutische Verwendung einer kosmetischen Zusammensetzung nach einem der Ansprüche 1 bis 8 zur Verbesserung der Straffheit der Haut und Reduzierung von Hautfalten.

## Claims

1. Cosmetic composition comprising dedifferentiated and elicited plant cells of Bougainvillaea in the form of vesicles, said vesicles comprising a plant membrane formed of a lipid bilayer delimiting an inner volume enclosing an encapsulated phase, said composition being **characterized in that** it further comprises at least one oligomer of hyaluronic acid of molecular weight lower than 10 kDa, and **in that** a saffron extract is contained in the encapsulated phase of the vesicles.

2. The composition according to claim 1, **characterized in that** it is a cosmetic composition for topical application.

3. The composition according to claim 1 or 2, **characterized in that** the amount of saffron extract contained in the encapsulated phase varies from 0.01 to 5 % by weight relative to the total weight of the dedifferentiated and elicited Bougainvillea cells.

4. The composition according to any of the preceding claims, **characterized in that** it comprises 0,01 % to 10 % by weight of a suspension of dedifferentiated and elicited Bougainvillea plant cells encapsulating a saffron extract in glycerine, relative to the total weight of said composition.

5. The composition according to one of the preceding claims, **characterized in that** the oligomer(s) of hyaluronic acid of molecular weight lower than 10 kDa meet the formula (C₁₄H₂₁NO₁₁) where n is an integer lower than 25 designating the degree of polymerisation.

6. The composition according to any of the preceding claims, **characterized in that** the oligomer concentration of hyaluronic acid of molecular weight lower than 10 kDa varies from 0,01 % to 1 % by weight relative to the total weight of said composition.

7. The composition according to any of the preceding claims, **characterized in that** it further comprises one or more active ingredients selected from among plankton extracts, maca extract, poppy petal extract, a combination of extracts of Anchusa, poppy and Passiflora, an extract of mimosa seeds, marigold petal extract, barbitamao extract, Kigelia africana extract and oat extract.

8. The composition according to any of the preceding claims, **characterized in that** it is in the form of a gel, emulsion, biphase oil-in-water or water-in-oil emulsion, body oil, mask, ointment, unguent or concentrated solution.

9. Non-therapeutic cosmetic method for skin care, **characterized in that** it consists of applying to the skin regions concerned at least one cosmetic composition such as defined in any of claims 1 to 8.

10. Non-therapeutic use of a cosmetic composition such as defined in any of claims 1 to 8 for skin care.

11. Non-therapeutic use of a cosmetic composition such as defined in any of claims 1 to 8, to improve skin firmness and reduce skin wrinkles.
